# FASCICULE DE BREVET EUROPEEN

(11) **EP 4 498 990 B1**
(45) Date de publication et mention de la délivrance du brevet: **20.05.2026**
(21) Numéro de dépôt: 23715091.7
(22) Date de dépôt: 24.03.2023
(51) Int. Cl.: A61F 2/52, A61F 2/30

(54) **PROCÉDÉ DE MODÉLISATION D'UNE PROTHÈSE ANATOMIQUE PAR CONCEPTION TRIDIMENSIONNELLE ASSISTÉE PAR ORDINATEUR,**
VERFAHREN ZUR HERSTELLUNG EINER ANATOMISCHEN PROTHESE DURCH COMPUTERGESTÜTZTES DREIDIMENSIONALES DESIGN
METHOD FOR PRODUCING AN ANATOMICAL PROSTHESIS BY COMPUTER-AIDED THREE-DIMENSIONAL DESIGN

(30) Priorité: 24.03.2022 FR 2202659
(43) Date de publication de la demande: 05.02.2025
(73) Titulaire: Atelier J'M Sasu, 75017 Paris (FR)
(72) Inventeur: MONTENERO, Julien, 92300 Levallois-Perret (FR)
(74) Mandataire: Spigarelli, Alfred
(86) Numéro de dépôt international: PCT/EP2023/057745
(87) Numéro de publication internationale: WO 2023/180573

(56) Documents cités:
- DE-A1- 102018 006 839
- GARSON ET AL: "Apport de l'imagerie 3D a la chirurgie mammaire : etude preliminaire", ANNALES DE CHIRURGIE PLASTIQUE ESTHETIQUE, EXPANSION SCIENTIFIQUE FRANCAISE, PARIS, FR, vol. 50, no. 4, 1 August 2005 (2005-08-01), pages 296 - 308, XP005010916, ISSN: 0294-1260

## Description

### DOMAINE TECHNIQUE DE L'INVENTION

L'invention concerne, de façon générale, le domaine technique des prothèses anatomiques destinées à réparer, restaurer ou rétablir des parties anatomiques pour des patients qui ont perdu un membre ou une partie de leur corps afin de leur redonner une apparence humaine dite normale.

Elle concerne en outre les procédés de production des prothèses anatomiques en matière synthétique souple ou composite avec des parties souples et rigides.

L'invention s'applique, en particulier, aux épithèses, aux prothèses faciales, aux prothèses maxillo-faciales, et aux prothèses mammaires externes à insérer dans une poche d'un sous-vêtement ou à plaquer directement, par exemple, par adhésif médical ou par des feuilles auto-adhésives, sur la peau après guérison complète à la suite d'une mastectomie totale ou partielle.

### ÉTAT DE LA TECHNIQUE ANTÉRIEURE

Des prothèses en matières synthétiques sont connues et sont généralement délivrées après mutilation ou ablation par acte chirurgical à la suite des accidents ou dans les pathologies cancéreuses invasives nécessitant une exégèse large ou limitée. En effet, dans ces conditions les patients ont besoin d'une prothèse en substitution de la partie anatomique retirée afin de retrouver leur intégrité physique et une apparence la plus identique possible à leur apparence d'avant et pouvoir ainsi poursuivre une vie sociale normale.

Le processus consiste à remplacer par une prothèse faite sur mesure le plus souvent en silicone les parties anatomiques retirées à la suite de toute circonstance nécessitant la prescription d'une prothèse anatomique, par exemple à la suite d'un cancer, d'un traumatisme, pour les grands brûlés, à la suite des maladies congénitales, ou à la suite de mutilations consécutives à des agressions, des attentats et pour les victimes de guerre.

En France, 20.000 femmes, et, au niveau mondial, 720.000 femmes, subissent chaque année des actes chirurgicaux lourds avec des conséquences irréversibles à la suite du diagnostic d'un cancer invasif du sein. Ces pathologies cancéreuses nécessitent dans la plupart des cas une exérèse plus ou moins large, appelée mastectomie, d'un sein ou parfois des deux. On parle alors de mastectomie bilatérale. Dans certains cas, l'exérèse sera limitée à une zone ciblée dans laquelle s'est développée une tumeur. Cette zone est le plus souvent située au niveau du derrière de la zone mamelonnaire. Cet acte chirurgical limité est appelé tumorectomie. Les conséquences psychologiques de ces mutilations impactent sévèrement les patientes et elles peuvent faire appel à différentes techniques afin de retrouver leur apparence d'avant l'acte chirurgical et de pouvoir reprendre une vie sociale normale.

Parfois, une chirurgie réparatrice sera mise en œuvre, mais par exemple dans le cas d'une mastectomie partielle ou d'une tumorectomie le volume original du sein pourra être restitué, mais ni le mamelon ni l'aréole qui sont des parties importantes de l'aspect d'un sein. Certaines patientes refusent une reconstruction ou cette reconstruction n'est pas possible médicalement.

Il existe des prothèses standards présentant différents volumes de tailles prédéterminées et de différents colories dans une gamme standard. Du fait que ces prothèses sont standards, elles ne seront jamais exactement identiques à la morphologie de la patiente avant son opération chirurgicale. De plus, ces prothèses standards sont exclusivement destinées à être insérées dans une poche d'un sous-vêtement adapté à cet usage.

Afin que le plus grand nombre de patients ayant subi une mutilation ou une exérèse chirurgicale, par exemple une mastectomie, puisse se reconstruire psychologiquement et physiquement à la suite de ce traumatisme, il existe un besoin pour une prothèse mammaire, ou autre, qui reproduit à l'identique la partie anatomique retirée pour la remplacer, mais qui soit abordable pour la majorité des patients. A cette fin, un procédé de production de prothèses anatomiques identiques à la partie anatomique retirée est nécessaire pour se substituer aux prothèses standards existantes.

La demande de brevet DE102018006839 A1 divulgue un procédé de réalisation à partir de relevés numériques de la partie du corps qui doit subir l'ablation avant et après l'intervention chirurgicale. Ces relevés numériques pré- et postopératoire sont obtenus à partir des fichiers DICOM réalisés par les techniques usuelles d'imagerie médical, telles que la mammographie, la sonographie, la tomographie, la résonance magnétique nucléaire, le scanner médical... Ces fichiers DICOM font partie du dossier médical de la patiente et qui ont été réalisés par des équipes médicales avant et après l'intervention chirurgicale. Ensuite, les fichiers DICOM sont transcrits en fichier STL pour être exploitables par une imprimante 3D. La précision des fichiers obtenus par les techniques d'imagerie médicales ne permet pas d'obtenir la précision suffisante pour un bon rendu visuel de la prothèse. Ce document ne montre ni un premier relevé volumique numérique qui est obtenu par numérisation tridimensionnelle réalisé par scan surfacique tridimensionnel d'une enveloppe du volume d'au moins une partie du corps d'un patient avant ablation d'une partie anatomique à remplacer par ladite prothèse anatomique, ni un deuxième relevé volumique numérique obtenu par numérisation tridimensionnelle réalisé par scan surfacique tridimensionnel d'une enveloppe du volume de la même dite au moins une partie du corps du patient après ablation de la partie anatomique à remplacer par ladite prothèse anatomique

La présente invention a pour but de remédier à tout ou partie des inconvénients de l'état de la technique en proposant notamment d'utiliser et d'exploiter les technologies actuelles de numérisation tridimensionnelle, de conception 3D assistée par ordinateur, et d'impression 3D pour former une chaîne de technologies numériques pour réaliser des prothèses personnalisées à chaque patient, afin de reproduire sa morphologie d'origine.

Pour ce faire, il est proposé, selon un premier aspect de l'invention, un procédé de modélisation d'une prothèse anatomique par conception tridimensionnelle assistée par ordinateur tel que défini dans les revendications 1 et 2.

Selon un deuxième aspect de l'invention, il est proposé un procédé de production d'une prothèse anatomique en matière synthétique souple par conception tridimensionnelle assistée par ordinateur tel que défini dans les revendications 3 à 11.

Selon un troisième aspect de l'invention, il est proposé un produit programme d'ordinateur tel que défini dans la revendication 12.

Selon un quatrième aspect de l'invention, il est proposé support de données lisible par ordinateur, sur lequel est enregistré le produit programme d'ordinateur.

Selon un cinquième aspect de l'invention, il est proposé un signal d'un support de données, portant le produit programme d'ordinateur.

D'autres caractéristiques et avantages de l'invention sont mis en évidence par la description ci-après d'exemples non limitatifs de réalisation des différents aspects de l'invention.

### BRÈVE DESCRIPTION DES FIGURES

La description se réfère aux figures annexées qui sont aussi données à titre d'exemples de réalisation non limitatifs de l'invention :
La figure 1a montre une représentation d'une partie du corps d'une patiente avant mastectomie ;
La figure 1b montre une représentation d'une partie du corps d'une patiente après mastectomie ;
La figure 1c montre une représentation d'une numérisation volumique avant mastectomie ;
La figure 2a illustre le dispositif permettant une superposition des numérisations volumiques avant et après mastectomie ;
La figure 2b illustre le relevé tomographique du sein à retirer ;
La figure 3 illustre un premier procédé de production de la prothèse anatomique ;
La figure 4 montre une vue en coupe d'une prothèse mammaire selon un premier mode de réalisation ;
La figure 5 illustre un deuxième procédé de production de la prothèse anatomique ;
La figure 6 montre une vue en coupe d'une prothèse mammaire selon un deuxième mode de réalisation ;
La figure 7 montre une prothèse mammaire provisoire ;

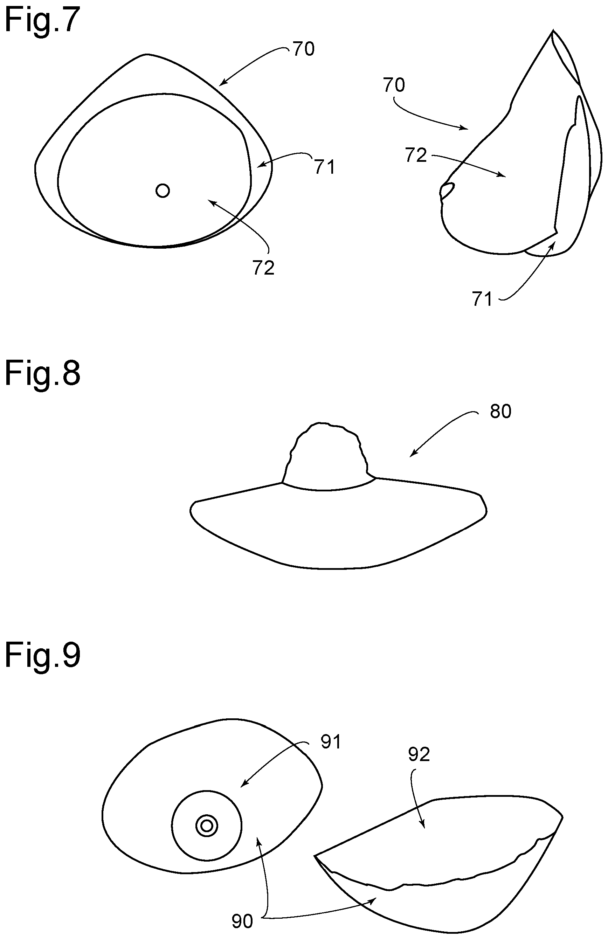

La figure 7 montre une prothèse mamelonnaire et aréolaire ; et
La figure 9 montre une prothèse mammaire recouvrante.

Pour plus de clarté, les éléments identiques ou similaires sont repérés par des signes de référence identiques sur l'ensemble des figures.

### DESCRIPTION DÉTAILLÉE D'UN MODE DE RÉALISATION

Dans la suite de la description, l'invention sera décrite principalement en référence aux prothèses mammaires délivrée après une mastectomie totale ou partielle d'un sein ou bilatérale. Néanmoins, les principes et techniques mis en œuvre, et décrits ci-dessous s'appliquent mutatis mutandis à tout type de prothèse dans les situations indiquées ci-dessus. Ainsi, l'invention peut s'appliquer également aux prothèses de substitution d'un membre après amputation, aux épithèses, aux prothèses faciales, at aux prothèses maxillo-faciales.

La solution proposée par l'invention consiste à représenter et à reproduire à l'identique la partie anatomique qui est retirée, par exemple un sein après mastectomie, afin de la remplacer. Au-delà de la technicité des actes compris dans le procédé de fabrication d'une telle prothèse anatomique, certaines étapes ont également pour but d'écouter et d'appréhender les traumatismes subis par les patients.

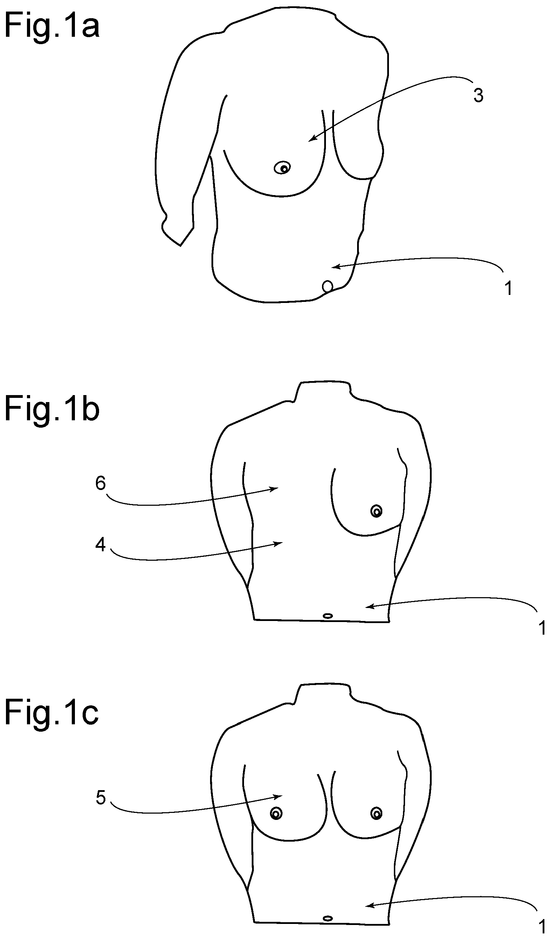

Comme représenté à la Figure 1a [Fig. 1a], le procédé de production de la prothèse anatomique commence par un premier relevé surfacique tridimensionnel de la zone du corps de la patiente dans laquelle se situe la partie anatomique à retirer, ici un sein. Ce premier relevé surfacique tridimensionnel a lieu lors d'un premier rendez-vous avec la patiente au cabinet du prothésiste à l'occasion duquel différentes propositions de prothèses lui sont présentées à l'aide d'images de synthèse produite sur la base du premier relevé surfacique tridimensionnel, par exemple des simulations de prothèses temporaires et définitives. Ce relevé surfacique tridimensionnel est réalisé par scanneur surfacique 3D ou par toute autre technique de numérisation tridimensionnelle d'un volume qui retranscrit les volumes anatomiques scannés en fichier numérique. Après l'acte chirurgical, dans l'exemple de mise en œuvre de l'invention décrit ci-dessous une mastectomie, une nouvelle intervention est réalisée pour procéder à un deuxième relevé surfacique tridimensionnel.

Le scanneur surfacique tridimensionnel ou scanneur 3D permet de réaliser des scans 3D par l'utilisation d'une technique qui permet de capturer la forme d'un objet ou d'une partie anatomique. Le résultat obtenu est un fichier 3D informatique qui peut être enregistré, édité et même imprimé en 3D. Plusieurs technologies permettent de scanner en 3D des objets, environnements ou personnes. Le scanneur 3D va analyser un objet et produire un nuage de points de la surface de l'objet à scanner afin d'en numériser l'enveloppe extérieure c'est-à-dire son volume. Ce scan 3D est traité par un loigiciel 3D pour obtenir une reproduction précise de l'objet scanné. Les scanneur 3D utilisent pour la plupart la technologie laser à triangulation ou la lumière structurée.

Les données résultant d'un scan 3D, c'est-à-dire les coordonnées des points mesurés appartenant à l'enveloppe de l'objet sont stockées dans un fichier au format STL. ,OBJ ,PLY, etc. Les scanneurs 3D sans contact direct représentent une solution technique non-invasive permettant d'enregistrer avec exactitude des parties anatomiques d'un corps sans en altérer les tissus afin d'en capturer sous forme numérique la forme exacte de l'enveloppe du volume de la partie anatomique scannée. DE plus ces fichiers numériques sont facilement transposables pour obtenir in fine une impression 3D de l'objet ou de la partie anatomique scannée.

Des exemples de scanneurs surfaciques 3D utilisables pour les application décrites ci-dessous sont les scanners a lumières struturées comme ceux de la société ARTEC (ARTEC EVA - LEO et SPIDER ). Des scaners de type smartphone ou tablette peuvent etre également utilisés. Ils permettent tous d'enregistrer l'enveloppe du volume de la partie anatomique scannée et d'en restituer un fichier STL, OBJ, PlY... Ces fichiers numériques modélisent selon les besoins un volume anatomique pour permettre ensuite différents traitements numériques tels que leur enrichissement par des données complémentaires ou l'mpression 3D.Lors du premier rendez-vous avec la patiente avant la mastectomie, un relevé des couleurs de la peau du sein à remplacer par la prothèse anatomique est effectué en plus du premier relevé tridimensionnel. Ce relevé des couleurs de peau est effectué à l'aide, par exemple, d'un spectrophotomètre, tel qu'un Spectroshade ^{®}, ou d'un colorimètre, tel qu'un Datacolor Coloreader EZ ^{®}. Ce relevé des couleurs permet de réaliser des prises de mesure des couleurs de peau permettant d'enregistrer les carnations de la partie anatomique à retirer afin que la prothèse anatomique les reproduise fidèlement. À cette occasion, des prises de mesures photographiques centimétriques sont également réalisées pour enregistrer tous les détails apparents à la surface du sein à retirer, comme des taches de rousseur, des veines, etc.

Comme illustré par la figure 1a [Fig. 1a], chaque fois que c'est possible, un premier relevé volumique numérique d'une partie supérieure du corps de la patiente 1 est effectué avec optionnellement les autres prises de mesure colorimétriques et photographiques indiquées ci-dessus. L'objectif de ce premier relevé volumique numérique est d'obtenir une image tridimensionnelle de l'enveloppe du volume des parties anatomiques par scanneur surfacique 3D pour reproduire à l'identique les volumes anatomiques avant toute transformation de cette partie du corps de la patiente 1 à la suite d'une mastectomie.

Ainsi, la première intervention consiste à réaliser une première empreinte numérique 3D d'au moins une partie du corps de la patiente afin d'enregistrer tous les détails anatomiques existant avant la mastectomie. Cette première empreinte numérique 3D permet d'obtenir le premier relevé volumique numérique 3. Dans le cas d'une mastectomie d'un sein, le relevé volumique numérique représente la transcription numérique de l'enveloppe de la partie volumétrique exacte du sein avec une précision d'environ 100 microns. Ainsi, l'enveloppe des volumes exacts d'une aréole et d'un mamelon de la patiente seront relevés dans le but de retranscrire méthodiquement les volumes réels qu'une prothèse anatomique, ici une prothèse mammaire, doit reproduire.

La figure 1b [Fig. 1b] illustre une deuxième phase du procédé qui a lieu après la mastectomie. Lors d'un deuxième rendez-vous avec la patiente après la mastectomie au cabinet du prothésiste, un deuxième relevé surfacique tridimensionnel de la même partie supérieure du corps de la patiente 1 après ablation du sein est réalisé. Ce deuxième relevé surfacique tridimensionnel est effectué selon la même technique que le premier et permet de réaliser une deuxième empreinte numérique 3D de la partie supérieure du corps de la patiente 1 après mastectomie sous la forme d'un deuxième relevé volumique numérique 4. L'objectif de ce deuxième relevé surfacique tridimensionnel est d'obtenir une numérisation tridimensionnelle de l'enveloppe du volume du corps, quand l'ablation a eu lieu. En particulier, le deuxième relevé volumique numérique 4 permet d'enregistrer la topographie de la partie du corps de la patiente qui a subi la mastectomie, et qui contient une cicatrice 6 consécutive à la mastectomie.

Ensuite, comme illustré à la figure 1c [Fig. 1c] le premier et le deuxième relevé volumique numérique 3 et 4 sont superposés par « matching » numérique des deux fichiers. On obtient ainsi par transparence une représentation volumique numérique 5 de l'enveloppe du volume de la partie anatomique retirée qui doit être remplacée par la prothèse anatomique. Ainsi, la prothèse anatomique à concevoir pourra être obtenue grâce à un logiciel de conception tridimensionnelle assistée par ordinateur qui formalise l'enveloppe du volume de la partie anatomique manquante à la suite de l'acte chirurgical. Ce logiciel de CAO tridimensionnelle utilise les fichiers du premier et du deuxième relevé volumique numérique 3 et 4 pour transposer les données contenues dans ces fichiers représentant les volumes scannés pour obtenir la représentation volumique numérique 5 illustrée à la figure 1c [Fig. 1c] sur la base de laquelle l'enveloppe du volume d'une pièce anatomique 3D peut-être dessinée et ses volumes reproduits par imprimante 3D comme décrit dans la suite.

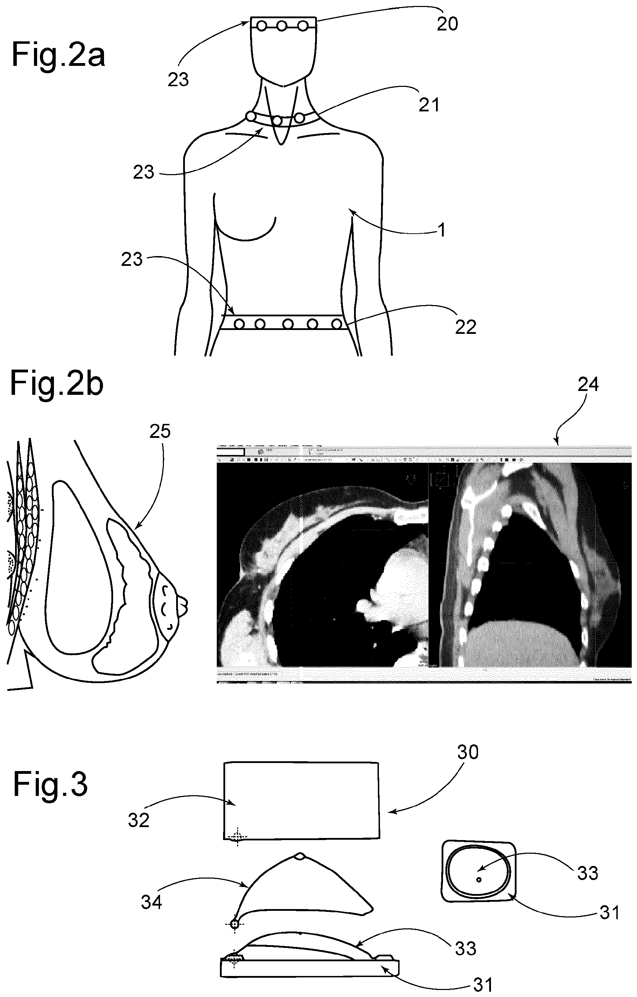

Pour permettre la superposition du premier et du deuxième relevé volumique numérique 3 et 4 et leur traitement par le logiciel de CAO tridimensionnel mentionné ci-dessus, des repères de positionnement doivent être restitués par les relevés volumiques tridimensionnels pré et postopératoires. Une des solutions possibles pour assurer ce positionnement des deux relevés consécutifs est illustrée à la figure 2a [Fig. 2a]. Dans cette solution, le premier et le deuxième relevé volumique numérique 3 et 4 sont effectués avec des repères positionnés sur la partie supérieure du corps de la patiente 1 sur laquelle sont effectués les relevés volumiques tridimensionnels. Dans cet exemple de mise en œuvre de cette partie de l'invention, ces repères sont placés sur la patiente avant de procéder au premier et au deuxième relevé volumique numérique 3 et 4. Ils se présentent, par exemple, sous la forme d'un serre-tête 20, d'un collier 21 et d'une ceinture 22. Chacun ces éléments de repérage présente des repères statiques 23. Par exemple, le serre-tête et le collier présentent chacun trois repères statiques 23, et la ceinture en présente cinq. Ces repères statiques 23 permettent une superposition parfaite du premier et du deuxième relevé volumique numérique 3 et 4 et le traitement des fichiers correspondants par le logiciel de CAO tridimensionnel permet d'obtenir la représentation volumique numérique 5 qui reproduit avec un très haut niveau de précision la partie anatomique retirée que la prothèse anatomique doit remplacer.

Comme illustré à la figure 2b [Fig. 2b], un relevé tomo-densitométrique 24 réalisé avant la mastectomie est également pris en compte dans le procédé de l'invention pour obtenir une reproduction encore plus fidèle de la partie anatomique à remplacer par la prothèse anatomique. Le relevé tomo-densitométrique 24 est réalisé, par exemple, avec un scanneur de tomodensitométrie ou par IRM (Imagerie par résonance magnétique). De préférence, ce relevé tomo-densitométrique 24 sera traité par un logiciel de transmission densitométrique pour obtenir un relevé tomographique numérique 25. Grâce à ce relevé tomographique numérique 25, la répartition de la densité tissulaire dans le sein à retirer est reproduite à l'identique dans la prothèse anatomique destinée à le remplacer après la mastectomie. Avantageusement, le relevé tomo-densitométrique 24 est réalisé lors du premier rendez-vous avec la patiente avec les éléments de repérage 20 à 22 illustrés à la figure 2a [Fig. 2a] positionnés sur la partie du corps de la patiente 1.

Dans certains cas particuliers, par exemple lorsque la phase préliminaire de prise de relevé tridimensionnel préopératoire illustrée à la figure 1a [Fig. 1a] n'a pas pu être faite, ou dans les cas de mastectomie bilatérale, il est possible de réaliser une simulation de l'enveloppe du volume formé par la morphologie de la patiente avant la mastectomie. En cas de mastectomie unilatérale, un fichier volumique tridimensionnel de l'anatomie de la patiente avant la mastectomie peut-être réalisé par transcription symétrique de l'enveloppe du volume de la partie de l'anatomie de la patiente non affectée par la mastectomie. Ce fichier tridimensionnel obtenu par symétrie pourra être utilisé à la place du premier relevé volumique numérique 3. En cas de mastectomie bilatérale, un fichier volumique tridimensionnel de l'anatomie de la patiente avant la mastectomie peut-être réalisé sur la base d'un ensemble de mesures standards préenregistrées correspondant à l'enveloppe du volume de la morphologie de la patiente. Dans cette situation, le fichier tridimensionnel obtenu par reconstruction virtuelle sur la base de mesures standards est utilisé à la place du premier relevé volumique numérique 3 après validation par la patiente.

Alternativement, il est possible de superposer des représentations volumiques 5 standards avec le deuxième relevé volumique numérique 4 de la patiente afin de définir la représentation volumique numérique qui correspond le mieux à l'anatomie de la patiente, et après « matching » des fichiers numériques correspondants, de formaliser par conception 3D assistée par ordinateur des prothèses appropriées à l'anatomie de la patiente sans utiliser le premier relevé volumique numérique 3. Dans le cas de l'ablation d'un seul sein sans que le premier relevé volumique numérique 3 n'ait pu être réalisé, la représentation volumique numérique 5 de la prothèse anatomique à réaliser est effectuée par CAO 3D sur la base du sein non retiré. Ainsi le procédé de l'invention permet d'appréhender toutes les situations et tous les challenges de la reconstruction postopératoire par prothèse externe et peut aussi s'appliquer aux situations dans lesquelles le premier relevé volumique numérique ne peut pas être réalisé, par exemple dans les cas de mutilation accidentelle.

Comme indiqué ci-dessus, dans le procédé de production de prothèses anatomique décrit, ici des prothèses mammaires, les différents fichiers numériques résultant des différents relevés réalisés sur la patiente avant et après l'acte chirurgical sont transposés et formatés pour permettre une fabrication additive, aussi appelée impression 3D, selon par exemple un procédé SLA, FDM ou SLS, d'un moule ou directement de la prothèse anatomique qu'elle soit temporaire, prototypique ou définitive comme expliqué dans la suite.

La figure 3 [Fig. 3] montre un moule 30 et sa constitution. Dans ce premier exemple de fabrication de prothèse anatomique, le fichier numérique correspondant à la représentation volumique numérique 5 est transposé et formaté pour pouvoir être utilisé par une imprimante 3D afin de produire par fabrication additive un moule dans lequel la matière synthétique souple constitutive de la prothèse anatomique, par exemple un silicone biomédical, sera injectée. Les propriétés mécaniques que doit présenter le moule 30 nécessitent d'utiliser des matériaux synthétiques rigides tels que les ABS, PLA, PP, nylon, etc. Pour ce faire, le moule 30 peut-être obtenu par un des trois types d'impression 3D, c'est-à-dire FDM, SLA ou SLS.

La production de prothèse anatomique par injection de silicone biomédical dans un moule 30 obtenu par impression 3D à partir de la représentation volumique numérique 5, permet d'obtenir une prothèse anatomique reproduisant à l'identique la partie anatomique retirée. Le silicone qui sera injecté ou coulé dans le moule 30 sera teinté dans la masse afin de reproduire fidèlement la carnation de la partie anatomique retirée sur la base du relevé colorimétrique numérique réalisé sur la patiente.

Le moule 30 peut être réalisé en deux parties, une base 31 et un volume d'injection 32 pour produire une prothèse anatomique en silicone versé ou injecté dans le moule 30. Dans ce cas, la densité de la prothèse anatomique sera définie à partir du relevé tomographique numérique 25. La densité de la prothèse anatomique peut-être définie comme la densité moyenne des densités enregistrées dans le relevé tomographique numérique 25.

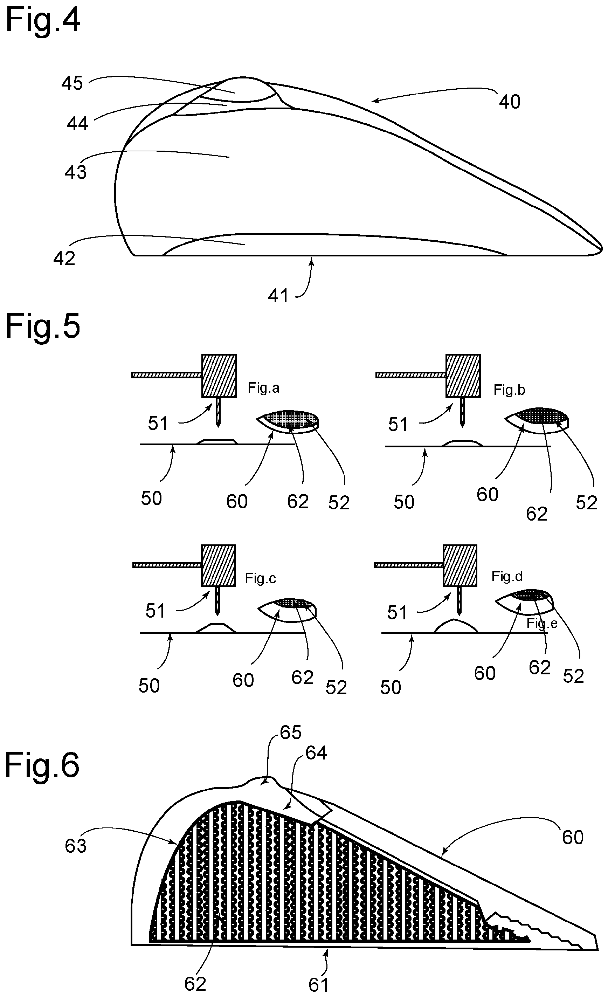

La figure 3 [Fig. 3] représente un moule 30 en plus de deux parties qui permettent de produire une prothèse anatomique en silicone à pluridensité. Comme expliqué ci-dessus, le moule 30 présente une base de moule 31 sur laquelle est formée en protubérance une empreinte de la cicatrice 33 qui représente fidèlement la topographie de la zone du corps de la patiente dans laquelle se trouve la cicatrice 6 telle que numérisée dans la représentation volumique numérique 5 à partir du deuxième relevé volumique numérique 4 réalisé après la mastectomie sur la partie du corps de la patiente 1. Ainsi, comme illustrée par la figure 4, une prothèse anatomique moulée à pluridensité 40 présente une base moulée 41 destinée à être mise en contact avec la peau de la patiente et à épouser intimement la topographie de la zone contenant la cicatrice 6 après le délai nécessaire pour la guérison complète postopératoire et après la fin de la radiothérapie ou de la chimiothérapie consécutive à la mastectomie.

La prothèse anatomique moulée à pluridensité 40 comprend différentes zones dans lesquelles des silicones de différentes structures ont été injectés dans le moule 30 suivant les densités transposées du relevé tomographique numérique 25. La prothèse anatomique moulée à pluridensité 40 illustrée à la figure 4 [Fig.4] comprend une zone inférieure 42 sur laquelle l'empreinte de la cicatrice 33 sur la base du moule 31 aura reproduit en négatif la topographie de la zone du corps de la patiente dans laquelle se trouve la cicatrice 6. La prothèse anatomique moulée à pluridensité 40 comprend également une zone interne 43, une zone aréolaire 44 et une zone mamelonnaire 45. Chacune de ces zones 41 à 45 de la prothèse anatomique moulée à pluridensité 40 présente une densité spécifique déterminée à partir de la densité de la zone respective du sein qui a été retiré à partir des mesures sensitométriques enregistrées dans le relevé tomographique numérique 25.

Pour réaliser la prothèse anatomique moulée à pluridensité 40, on utilise un moule 30 dans lequel une partie intérieure de moule 34 peut être positionnée sur la base du moule 31. Dans ce cas, plusieurs parties intérieures de moule 34 sont nécessaires pour obtenir une prothèse anatomique moulée à pluridensité 40. Par exemple, pour réaliser la prothèse anatomique moulée à pluridensité 40 montrée à la figure 4 [Fig. 4], on commencera par positionner une première partie intérieure de moule 34, ne laissant libre dans le volume du moule 32 que le volume correspondant à la zone mamelonnaire 45 dans lequel un silicone présentant la densité de la zone mamelonnaire sera injecté. Dans une deuxième étape, la première partie intérieure de moule 34 est remplacée par une deuxième partie intérieure de moule dont la forme permet d'injecter un silicone dont la densité correspond à la densité de la zone aréolaire 44 afin de réaliser la zone aréolaire 44. Une troisième partie intérieure de moule permet d'injecter un silicone de densité voulue pour réaliser la zone intérieure 43. La zone inférieure 42 est réalisée en dernier sans insertion d'une partie intérieure de moule 34.

Après réalisation de la prothèse anatomique moulée à monodensité ou à pluridensité 40, il est possible de procéder à une étape de finition en appliquant un agent final de recouvrement permettant de reproduire tous les détails extérieurs enregistrés dans le relevé photographique centimétrique. L'agent de recouvrement utilisé pour cette étape de finition, peut-être un silicone médical présentant différentes pigmentations extrinsèques qui est appliqué, par exemple, avec un aérographe ou un pinceau.

La figure 5 [Fig. 5] illustre différentes étapes de la production d'une prothèse anatomique imprimée 60 par impression 3D, préférentiellement par impression 3D de type SLA ou FDM comme montré ici. La prothèse anatomique peut être obtenue directement par impression 3D en formatant le fichier de la représentation vomique numérique 5 pour qu'une imprimante 3D réalise une prothèse anatomique reproduisant à l'identique le volume de la partie anatomique retirée. Le silicone biomédical utilisé à cet effet peut-être teinté dans la masse à l'aide du relevé colorimétrique numérique de manière à ce que la prothèse anatomique reproduise également la carnation de la patiente.

Du fait de la complexité de la forme de la prothèse anatomique, l'impression 3D FDM ou SLA est particulièrement adaptée pour la fabrication des prothèses anatomiques, en particulier l'impression 3D SLA du fait qu'une base imprimée 61 peut devoir représenter en négatif la topographie de la zone du corps de la patiente dans laquelle se trouve la cicatrice 6 telle que numérisée dans la représentation volumique numérique 5.

L'impression 3D permet de réaliser aussi bien des prothèses anatomiques à monodensité ou pluridensité en faisant varier la structure interne de la prothèse anatomique selon les données du relevé tomographique numérique 25. La structure interne de la prothèse peut prendre différentes formes. La figure 5 montre une prothèse anatomique en cours d'impression présentant une structure en treillis 62, définie aussi par son appellation anglophone « lattice ». La base imprimée 61 de la prothèse anatomique repose sur un plateau d'impression 50 d'une imprimante 3D 51, par exemple de type FDM sur laquelle une base d'impression (non représentée) est positionnée qui peut représenter la topographie du site de la cicatrice 6 et sur laquelle sera imprimé le volume de la prothèse anatomique 60. La tête d'impression 52 apporte le filament qui est fondu aux emplacements où la matière doit être déposée couche par couche sur le plateau d'impression 50.

La figure 6 [Fig. 6] montre une prothèse anatomique imprimée 60 en coupe. Ainsi, la structure interne présente un lattice 62 réalisé par impression 3D dont la structure permet de reproduire les différentes mesures de densité enregistrées dans le relevé tomographique numérique. Une structure périphérique 63 reproduit la carnation enregistrée dans le relevé colorimétrique numérique 25 ainsi que la densité périphérique de la partie anatomique retirée. Il en va de même pour une zone aréolaire 64, et une zone mamelonnaire 65. La base imprimée 61 reproduit en négatif la topographie de la zone anatomique de la patiente comprenant la cicatrice 6 de manière à ce que la base imprimée épouse intimement la forme de la cicatrice 6 de la patiente.

L'impression 3D permet de réaliser directement une prothèse anatomique imprimée 60 reproduisant à l'identique tous les détails externes et internes du sein retiré, y compris ceux enregistrés lors du relevé photographique centimétrique. Optionnellement, les détails externes enregistrés dans le relevé photographique centrimétrique peuvent être réalisés lors d'une étape de finition, qui peut-être exécutée manuellement avec, par exemple, un aérographe pulvérisant des silicones biomédicaux teintés pour reproduire ces détails externes, ou un pinceau.

Par exemple, lorsque la patiente peut supporter une prothèse anatomique dite définitive, telle que la prothèse anatomique imprimée 60 décrite ci-dessus, celle-ci peut être imprimée de façon très réaliste à l'identique de la partie anatomique dont la forme a été numérisée dans la représentation volumique numérique 5. Le procédé de l'invention permet alors de générer des joints de contour 66 permettant à la prothèse d'être appliquée très intimement au corps de la patiente afin d'en rendre les contours quasiment invisibles grâce à la couleur et à la texture sur mesure de la prothèse anatomique imprimée 60. Le procédé d'obtention d'une prothèse anatomique par moulage décrit ci-dessus permet également d'obtenir une prothèse définitive de ce type.

Ainsi les modes de mise en œuvre du procédé de l'invention décrit ci-dessus permettent de retranscrire par impression 3D le volume, la peau, la carnation et la densité du sein. En effet, les masses internes relevées en densitométrie corporelle seront transposées par logiciel pour représenter les données relevées à l'intérieur du sein avant son ablation ou du sein controlatéral lorsque nécessaire.

Avant de réaliser la prothèse anatomique définitive décrite ci-dessus, il peut être opportun de produire un prototype en silicone ou en résine afin de valider le volume de la prothèse anatomique après visualisation du volume la représentant. L'étape de finition avec l'agent de recouvrement, et l'adaptation finale sur la patiente afin de retoucher et d'affiner toutes les caractéristiques possibles, notamment le joint de contour 66, peuvent ainsi être réalisées pendant ou après validation de la forme de la prothèse anatomique. La visualisation et la validation d'une prothèse anatomique prototypique sont particulièrement importantes lorsque le premier relevé volumique numérique 3 n'a pas pu être réalisé avant la mastectomie.

Avant de pouvoir bénéficier d'une prothèse anatomique définitive imprimée ou moulée, une prothèse temporaire est proposée aux patientes en phase de cicatrisation et durant les traitements postopératoires par radiothérapie ou chimiothérapie durant lesquelles les patientes ne peuvent pas supporter un contact de la prothèse avec la peau.

Néanmoins, pour que ces patientes puissent bénéficier rapidement après la mastectomie d'une prothèse individuelle correspondant à leur anatomie, les procédés de mise en œuvre de l'invention permettent de produire des prothèses anatomiques provisoires réalistes et adaptées à la morphologie de la patiente, mais utilisables dans les soutiens-gorge au lieu d'être collées par adhésif médical directement sur la peau de la patiente. Ces prothèses provisoires sont adaptées pour être reçues dans une poche textile prévue à cet effet dans les sous-vêtements de la patiente. Cette solution provisoire donne la possibilité aux patientes de pouvoir bénéficier de la technologie décrite ci-dessus et ainsi de conserver leur anatomie propre en comparaison aux solutions actuelles standards qui ne reproduisent pas à l'identique l'anatomie des patientes.

En phase de cicatrisation ou pendant les traitements de radiothérapie et de chimiothérapie, les patientes n'auront aucun problème à tolérer ces prothèses anatomiques provisoires en attendant de pouvoir tolérer la prothèse anatomique définitive intimement positionnée à même la peau et qui peut-être collée par un adhésif biomédical ou une feuille auto-adhésive pour une tenue de la prothèse directement sur le corps de la patiente.

La figure 7 [Fig. 7] montre une prothèse anatomique provisoire 70 produite par moulage ou par impression 3D comme décrit ci-dessus et illustré aux figures 3 et 5. Cette prothèse anatomique provisoire 70 est destinée à être intégrée dans une poche de sous-vêtements afin d'être portée notamment durant les mois qui suivent la mastectomie. La prothèse anatomique provisoire 70 est généralement délivrée environ deux mois après la mastectomie. Elle peut donc être réalisée en tant que modèle provisoire dans l'attente de la guérison postopératoire complète, ou en attente de la reconstruction par prothèse interne. La prothèse anatomique provisoire 70 peut également servir de prototype en vue de finaliser la prothèse anatomique définitive.

Au préalable, un patron 71 est sélectionné, par exemple sur la base de la représentation volumique numérique 5 parmi les patrons virtuels correspondant aux différentes tailles de poitrine existantes. Ensuite, un volume de prothèse provisoire 72 formalisé par la représentation volumique numérique 5 est fusionné numériquement au patron 71. Le résultat de cette fusion de fichiers numériques sert de base pour la réalisation de la prothèse anatomique provisoire 70 par moulage ou directement par impression 3D.

La prothèse anatomique provisoire 70 est portée dans un soutien-gorge adapté. Le volume de la prothèse provisoire 72 n'est pas en contact direct avec la peau de la patiente, car c'est le patron 71 qui sera en regard de la peau à l'intérieur du sous-vêtement. Ainsi la topographie de la base du volume de la prothèse provisoire 72 n'a pas besoin d'être adaptée à la forme de la cicatrice 6. Le volume de la prothèse provisoire 72 est injecté ou imprimé à partir d'un silicone biomédical à monodensité correspondant à la densité moyenne du sein retiré de chaque patiente, ou pluridensité correspondant aux données du relevé tomographique numérique 25.

De même, la carnation de la prothèse anatomique provisoire 70 est représentée par la teinte du silicone injecté ou versé dans le moule 30, ou utilisé pour l'impression 3D. Cette teinte peut-être individuelle ou sélectionnée parmi un choix de couleurs représentant les couleurs usuelles de peau sur la base du relevé colorimétrique numérique. Un agent final de recouvrement peut-être appliqué sur le volume de la prothèse provisoire 72 pour finaliser les aspects extérieurs de la prothèse anatomique provisoire 70.

De nos jours, les progrès dans le diagnostic des cancers permettent de plus en plus souvent d'éviter la mastectomie totale et de ne pratiquer qu'une mastectomie partielle ou une tumorectomie qui entraîne en général l'ablation du mamelon et de la zone aréolaire. De plus, les progrès des techniques chirurgicales permettent plus souvent une reconstruction par prothèse interne où néanmoins le mamelon est manquant. Ainsi, les parties mamelonnaire et aréolaire sont de plus en plus demandées, car elles sont des éléments parmi les plus importants pour le réalisme de toutes les prothèses mammaires.

La figure 8 [Fig. 8] montre une prothèse mamelonnaire et aréolaire 80, qui est prescrite à la suite d'une tumorectomie où le mamelon et l'aréole sont retirés, ou dans le cas où ils sont manquants à la suite d'une reconstruction chirurgicale par prothèse interne après une mastectomie. Ainsi, le procédé selon l'invention permet d'obtenir par moulage ou par impression 3D une prothèse mamelonnaire et aréolaire 80 conceptualisée et formalisée comme décrit ci-dessus de procéder à leur conceptualisation afin qu'elle soit intégrée dans l'anatomie restante de la patiente pour reproduire à l'identique son anatomie d'avant l'opération.

La figure 9 [Fig. 9] montre une prothèse mammaire recouvrante 90. À la suite d'une mastectomie partielle, par exemple l'ablation de la moitié d'un sein, ou en cas de mastectomie totale suivie d'une reconstruction par prothèse interne, la prothèse mammaire recouvrante 90 peut-être prescrite. La prothèse mammaire recouvrante 90 est réalisée par moulage ou par impression 3D selon le procédé de l'invention décrit ci-dessus afin de reproduire à l'identique la moitié du sein retiré. Une face externe 91 de la prothèse mammaire recouvrante 90 reproduit à l'identique le mamelon, l'aréole et les détails extérieurs de la moitié du sein retiré à l'aide des outils numériques décrits ci-dessus. Ces outils permettent également de réaliser une face interne 92 qui sera en contact intime avec la partie du sein restante et qui cachera la cicatrice 6. Pour cela, la face interne 92 peut reproduire en négatif la topographie de la zone du sein contenant la cicatrice 6.

L'invention permet donc de réaliser des prothèses anatomiques, telles que des prothèses mammaires définitive ou provisoire, en reproduisant par impression 3D directe ou par injection dans un moule réalisé par impression 3D. Dans les deux cas, l'enveloppe du volume obtenu par l'impression 3D est réalisée à l'identique de l'enveloppe du volume de la partie anatomique à retirer à partir d'une représentation volumique numérique 5 du volume de la partie anatomique retirée à remplacer par la prothèse anatomique 2. Cette représentation volumique numérique 5 est obtenue par CAO 3D en superposant un premier relevé volumique numérique 3 réalisé avant l'acte chirurgical ou une simulation 3D de la partie du corps de patient avant l'acte chirurgical, et un deuxième relevé volumique numérique 4 réalisé après l'acte chirurgical.

Pour aller plus loin dans le réalisme au touché de la prothèse anatomique 2, en particulier lorsqu'il s'agit d'une prothèse définitive, la répartition des densités à l'intérieur de la prothèse anatomique 2 ou de la structure interne de la prothèse anatomique est reproduite à partir du relevé tomographique numérique 25. Il en va de même pour le réalisme visuel de la prothèse anatomique 2 qui peut-être amélioré en reproduisant la carnation du patient à partir du relevé colorimétrique et/ou du relevé photographique centimétrique de la partie anatomique à retirer. Les différents sets de données numériques provenant du relevé tomographique, du relevé colorimétrique et/ou du relevé photocentimétrique peuvent compléter le premier relevé numérique volumique 3 ou la représentation volumique 5

Le développement des différents modes de réalisation du procédé de production de prothèses anatomiques a vocation a réparer physiologiquement et psychologiquement les patients amputés ou mutilés, notamment les patientes ayant subi une mastectomie ou une tumorectomie à la suite d'un cancer du sein.

Comme indiqué dans la description qui précède, les différents aspects de l'invention peuvent-être mis en œuvre pour produire des prothèses autres que les prothèses mammaires sans pour autant sortir du cadre de l'invention. Par exemple, le procédé de l'invention permet de produire une épithèse pour remplacer un nez ou une oreille reproduisant à l'identique, ou du moins de façon réaliste si la partie mutilée n'a pas pu être scannée avant, la densité des tissus qui la composent ainsi que des parties cartilagineuses. Selon un autre exemple, le procédé de l'invention permet de reproduire à l'identique un membre amputé en jouant sur les matières moulée ou injectée, en particulier leurs densités et rigidités. Ainsi, la prothèse anatomique reproduira fidèlement les volumes reproduisant les muscles aussi bien que ceux reproduisant l'ossature du membre amputé.

Naturellement, l'invention est décrite dans ce qui précède à titre d'exemple. Il est entendu que l'homme du métier est à même de réaliser différentes variantes de réalisation de l'invention et de la mettre en œuvre pour d'autres finalités.

## Revendications

1. Procédé de modélisation d'une prothèse anatomique (2) par conception tridimensionnelle assistée par ordinateur, comprenant les étapes suivantes:
- obtention des premières données de représentation volumique numérique (5) d'une enveloppe du volume de la prothèse anatomique par superposition :
- d'un premier relevé volumique numérique (3) obtenu par numérisation tridimensionnelle réalisé par scan surfacique tridimensionnel d'une enveloppe du volume d'au moins une partie du corps d'un patient (1) avant ablation d'une partie anatomique à remplacer par ladite prothèse anatomique (2), ou par simulation numérique de l'enveloppe du volume d'au moins une partie du corps d'un patient (1) avant ablation d'une partie anatomique à remplacer par ladite prothèse anatomique (2), et
- d'un deuxième relevé volumique numérique (4) obtenu par numérisation tridimensionnelle réalisé par scan surfacique tridimensionnel d'une enveloppe du volume de la même dite au moins une partie du corps du patient (1) après ablation de la partie anatomique à remplacer par ladite prothèse anatomique (2).

2. Procédé de modélisation d'une prothèse anatomique (2) par conception tridimensionnelle assistée par ordinateur selon la revendication 1, comprenant en outre l'une ou les étapes suivantes :
- obtention des deuxièmes données par relevé colorimétrique numérique et/ou par relevé photographique centimétrique de ladite partie anatomique avant son ablation ; et/ou
- obtention des troisièmes données par relevé tomographique numérique (25) de ladite partie anatomique avant son ablation.

3. Procédé de production d'une prothèse anatomique en matière synthétique souple par conception tridimensionnelle assistée par ordinateur sur la base du procédé de modélisation d'une prothèse anatomique (2) d'une des revendications 1 ou 2.

4. Procédé de production d'une prothèse anatomique en matière synthétique souple par conception tridimensionnelle assistée par ordinateur selon la revendication 3, l'aspect visuel extérieur de la prothèse anatomique (2) reproduisant à l'identique l'aspect visuel de la partie anatomique à remplacer par ladite prothèse anatomique (2) sur la base des deuxièmes données ; et/ou
- des troisièmes données,
lesdites deuxièmes données et/ou troisièmes données venant compléter lesdites premières données.

5. Procédé de production d'une prothèse anatomique en matière synthétique souple par conception tridimensionnelle assistée par ordinateur selon l'une des revendications 3 ou 4 dans lequel :
- une base de ladite prothèse anatomique orientée vers la peau du patient est fusionnée à un patron (70) dont la taille correspond à celle de ladite base de la prothèse anatomique ; ou
- une base (41 ; 61) de ladite prothèse anatomique (2) destinée à être en contact avec la peau du patient présente une topographie obtenue à partir dudit deuxième relevé volumique numérique (4) afin de correspondre à l'identique au profile d'une zone du corps du patient d'où a été retirée la partie anatomique à remplacer par ladite prothèse anatomique (2).

6. Procédé de production d'une prothèse anatomique en matière synthétique souple par conception tridimensionnelle assistée par ordinateur selon l'une des revendications 3 à 5, un moule (30) étant réalisé sur la base de ladite représentation volumique numérique (5), dans lequel la matière synthétique souple est injectée pour former ledit volume de ladite prothèse anatomique (2).

7. Procédé de production d'une prothèse anatomique en matière synthétique souple par conception tridimensionnelle assistée par ordinateur selon la revendication 6, le moule (30) étant réalisé par fabrication additive à partir de la représentation volumique numérique (5).

8. Procédé de production d'une prothèse anatomique en matière synthétique souple par conception tridimensionnelle assistée par ordinateur selon l'une des revendications 6 ou 7, la matière synthétique souple injectée dans le moule (30) présentant :
- une carnation correspondant au relevé colorimétrique numérique ; et/ou
- une densité correspondant à la densité moyenne du relevé tomographique numérique (25), ou une répartition de densité dérivée du relevé tomographique numérique (25).

9. Procédé de production d'une prothèse anatomique en matière synthétique souple par conception tridimensionnelle assistée par ordinateur selon l'une quelconque des revendications 3 à 8, une étape de finition permettant de reproduire après le moulage de la prothèse anatomique (2) la pigmentation des différentes zones du volume de ladite prothèse anatomique (2) à l'identique de la partie anatomique à remplacer par la prothèse anatomique (2) sur la base du relevé colorimétrique numérique, et/ou du relevé photographique centimétrique.

10. Procédé de production d'une prothèse anatomique en matière synthétique souple par conception tridimensionnelle assistée par ordinateur selon l'une des revendications 3 ou 4,la prothèse anatomique (2) étant réalisée par fabrication additive sur la base :
- desdites premières données afin que la forme du volume de ladite prothèse anatomique (2) soit identique à la forme de la partie anatomique à remplacer par ladite prothèse anatomique (2) ;
- desdites deuxièmes données afin que l'aspect visuel de ladite prothèse anatomique (2) soit identique à l'aspect visuel de la partie anatomique retirée ; et/ou
- desdites troisièmes données afin que la répartition de la densité dans ladite prothèse anatomique (2) soit identique à la répartition de la densité dans la partie anatomique à remplacer par ladite prothèse numérique (2).

11. Procédé de production d'une prothèse anatomique en matière synthétique souple par conception tridimensionnelle assistée par ordinateur selon l'une des revendications 3 à 10, la matière synthétique souple utilisée étant un silicone biomédical teinté dans la masse afin de reproduire les couleurs au ton et au niveau de saturation requis par le relevé colorimétrique numérique.

12. Produit programme d'ordinateur comprenant des instructions qui lorsqu'elles sont exécutées par un ordinateur conduisent celui-ci à mettre en oeuvre le procédé d'une des revendications 1 à 11.

13. Support de données lisible par ordinateur, sur lequel est enregistré le produit programme d'ordinateur selon la revendication 12.

14. Signal d'un support de données, portant le produit programme d'ordinateur selon la revendication 12.

## Patentansprüche

1. Verfahren zur Modellierung einer anatomischen Prothese (2) durch computergestütztes dreidimensionales Konstruieren, umfassend die folgenden Schritte:
- Erhalten von ersten Daten einer digitalen Volumendarstellung (5) einer Hülle des Volumens der anatomischen Prothese durch Überlagerung:
- einer durch dreidimensionale Digitalisierung erhaltenen ersten digitalen Volumenerfassung (3), die durch dreidimensionalen Oberflächenscan einer Hülle des Volumens mindestens eines Teils des Körpers eines Patienten (1) vor Ablation eines durch die anatomische Prothese (2) zu ersetzenden anatomischen Teils oder durch digitale Simulation der Hülle des Volumens mindestens eines Teils des Körpers eines Patienten (1) vor Ablation eines durch die anatomische Prothese (2) zu ersetzenden anatomischen Teils ausgeführt wurde, und
- einer durch dreidimensionale Digitalisierung erhaltenen zweiten digitalen Volumenerfassung (4), die durch dreidimensionalen Oberflächenscan einer Hülle des Volumens desselben mindestens eines Teils des Körpers des Patienten (1) nach Ablation des durch die anatomische Prothese (2) zu ersetzenden anatomischen Teils ausgeführt wurde.

2. Verfahren zur Modellierung einer anatomischen Prothese (2) durch computergestütztes dreidimensionales Konstruieren nach Anspruch 1, umfassend ferner einen oder mehrere der folgenden Schritte:
- Erhalten von zweiten Daten durch digitale kolorimetrische Erfassung und/oder durch zentimetrische fotografische Erfassung des anatomischen Teils vor seiner Ablation; und/oder
- Erhalten von dritten Daten durch digitale tomographische Erfassung (25) des anatomischen Teils vor seiner Ablation.

3. Verfahren zur Herstellung einer anatomischen Prothese aus flexiblem synthetischem Material durch computergestütztes dreidimensionales Konstruieren auf der Grundlage des Verfahrens zur Modellierung einer anatomischen Prothese (2) nach einem der Ansprüche 1 oder 2.

4. Verfahren zur Herstellung einer anatomischen Prothese aus flexiblem synthetischem Material durch computergestütztes dreidimensionales Konstruieren nach Anspruch 3, wobei das äußere visuelle Erscheinungsbild der anatomischen Prothese (2) das visuelle Erscheinungsbild des durch die anatomische Prothese (2) zu ersetzenden anatomischen Teils auf der Grundlage der zweiten Daten und/oder
- der dritten Daten identisch nachbildet,
wobei die zweiten Daten und/oder dritten Daten die ersten Daten ergänzen.

5. Verfahren zur Herstellung einer anatomischen Prothese aus flexiblem synthetischem Material durch computergestütztes dreidimensionales Konstruieren nach einem der Ansprüche 3 oder 4, wobei:
- eine zu der Haut des Patienten gerichtete Basis der anatomischen Prothese mit einer Schablone (70) verschmolzen wird, deren Größe derjenigen der Basis der anatomischen Prothese entspricht; oder
- eine Basis (41; 61) der anatomischen Prothese (2), die dazu bestimmt ist, mit der Haut des Patienten in Kontakt zu sein, eine Topographie aufweist, die ausgehend von der zweiten digitalen Volumenerfassung (4) erhalten wird, damit sie mit dem Profil einer Region des Körpers des Patienten, aus welcher der durch die anatomische Prothese (2) zu ersetzende anatomische Teil entfernt wurde, identisch ist.

6. Verfahren zur Herstellung einer anatomischen Prothese aus flexiblem synthetischem Material durch computergestütztes dreidimensionales Konstruieren nach einem der Ansprüche 3 bis 5, wobei eine Form (30) auf der Grundlage der digitalen Volumendarstellung (5) ausgeführt wird, in die das flexible synthetische Material eingespritzt wird, um das Volumen der anatomischen Prothese (2) zu bilden.

7. Verfahren zur Herstellung einer anatomischen Prothese aus flexiblem synthetischem Material durch computergestütztes dreidimensionales Konstruieren nach Anspruch 6, wobei die Form (30) durch additive Fertigung ausgehend von der digitalen Volumendarstellung (5) ausgeführt wird.

8. Verfahren zur Herstellung einer anatomischen Prothese aus flexiblem synthetischem Material durch computergestütztes dreidimensionales Konstruieren nach einem der Ansprüche 6 oder 7, wobei das in die Form (30) eingespritzte flexible synthetische Material aufweist:
- eine Hautfarbe, die der digitalen kolorimetrischen Erfassung entspricht; und/oder
- eine Dichte, die der durchschnittlichen Dichte der digitalen tomographischen Erfassung (25) entspricht, oder eine aus der digitalen tomographischen Erfassung (25) abgeleitete Dichteverteilung.

9. Verfahren zur Herstellung einer anatomischen Prothese aus flexiblem synthetischem Material durch computergestütztes dreidimensionales Konstruieren nach einem der Ansprüche 3 bis 8, wobei ein Endbearbeitungsschritt es ermöglicht, nach der Formung der anatomischen Prothese (2) die Pigmentierung der verschiedenen Regionen des Volumens der anatomischen Prothese (2) wie bei dem durch die anatomische Prothese (2) zu ersetzenden anatomischen Teil auf der Grundlage der digitalen kolorimetrischen Erfassung und/oder der zentimetrischen fotografischen Erfassung identisch nachzubilden.

10. Verfahren zur Herstellung einer anatomischen Prothese aus flexiblem synthetischem Material durch computergestütztes dreidimensionales Konstruieren nach einem der Ansprüche 3 oder 4, wobei die anatomische Prothese (2) durch additive Fertigung ausgeführt wird auf der Grundlage:
- der ersten Daten, damit die Form des Volumens der anatomischen Prothese (2) identisch mit der Form des durch die anatomische Prothese (2) zu ersetzenden anatomischen Teils ist;
- der zweiten Daten, damit das visuelle Erscheinungsbild der anatomischen Prothese (2) identisch mit dem visuellen Erscheinungsbild des entfernten anatomischen Teils ist; und/oder
- der dritten Daten, damit die Verteilung der Dichte in der anatomischen Prothese (2) identisch mit der Verteilung der Dichte in dem durch die digitale Prothese (2) zu ersetzenden anatomischen Teil ist.

11. Verfahren zur Herstellung einer anatomischen Prothese aus flexiblem synthetischem Material durch computergestütztes dreidimensionales Konstruieren nach einem der Ansprüche 3 bis 10, wobei das verwendete flexible synthetische Material ein biomedizinisches Silikon ist, das durchgefärbt ist, um die Farben mit dem durch die digitale kolorimetrische Erfassung geforderten Farbton und Sättigungsgrad nachzubilden.

12. Computerprogrammprodukt, das Anweisungen umfasst, die bei ihrer Ausführung durch einen Computer diesen veranlassen, das Verfahren nach einem der Ansprüche 1 bis 11 durchzuführen.

13. Computerlesbarer Datenträger, auf dem das Computerprogrammprodukt nach Anspruch 12 gespeichert ist.

14. Signal eines Datenträgers, der das Computerprogrammprodukt nach Anspruch 12 trägt.

## Claims

1. Method for modelling an anatomical prosthesis (2) via computer-aided three-dimensional design, comprising the following steps:
- obtaining first data regarding a digital volume representation (5) of an envelope of the volume of the anatomical prosthesis by superposition:
- of a first digital volume survey (3) obtained by three-dimensional digitization, said survey being taken via a three-dimensional surface scan of an envelope of the volume of at least one part of the body of a patient (1) before ablation of an anatomical part to be replaced by said anatomical prosthesis (2), or via digital simulation of the envelope of the volume of at least one part of the body of a patient (1) before ablation of an anatomical part to be replaced by said anatomical prosthesis (2), and
- of a second digital volume survey (4) obtained by three-dimensional digitization, said survey being taken via a three-dimensional surface scan of an envelope of the volume of the same said at least one part of the body of the patient (1) after ablation of the anatomical part to be replaced by said anatomical prosthesis (2).

2. Method for modelling an anatomical prosthesis (2) via computer-aided three-dimensional design according to Claim 1, further comprising one or more of the following steps:
- obtaining second data via a digital colorimetric survey and/or via a centimetre-scale photographic survey of said anatomical part before its ablation; and/or
- obtaining third data via a digital tomographic survey (25) of said anatomical part before its ablation.

3. Method for producing an anatomical prosthesis made of flexible synthetic material via computer-aided three-dimensional design based on the method for modelling an anatomical prosthesis (2) according to either of Claims 1 and 2.

4. Method for producing an anatomical prosthesis made of flexible synthetic material via computer-aided three-dimensional design according to Claim 3, wherein the external visual appearance of the anatomical prosthesis (2) identically reproduces the visual appearance of the anatomical part to be replaced by said anatomical prosthesis (2) based on the second data; and/or
- third data,
said second data and/or third data complementing said first data.

5. Method for producing an anatomical prosthesis made of flexible synthetic material via computer-aided three-dimensional design according to either of Claims 3 and 4, wherein:
- a base of said anatomical prosthesis oriented towards the skin of the patient is fused to a template (70) the size of which corresponds to that of said base of the anatomical prosthesis; or
- a base (41; 61) of said anatomical prosthesis (2) intended to make contact with the skin of the patient has a topography obtained from said second digital volume survey (4) in order to correspond identically to the profile of a region of the body of the patient from which was removed the anatomical part to be replaced by said anatomical prosthesis (2).

6. Method for producing an anatomical prosthesis made of flexible synthetic material via computer-aided three-dimensional design according to any of Claims 3 to 5, wherein a mould (30) is produced based on said digital volume representation (5), into which mould the flexible synthetic material is injected to form said volume of said anatomical prosthesis (2).

7. Method for producing an anatomical prosthesis made of flexible synthetic material via computer-aided three-dimensional design according to Claim 6, wherein the mould (30) is produced by additive manufacturing from the digital volume representation (5).

8. Method for producing an anatomical prosthesis made of flexible synthetic material via computer-aided three-dimensional design according to either of Claims 6 and 7, wherein the flexible synthetic material injected into the mould (30) has:
- a complexion corresponding to the digital colorimetric survey; and/or
- a density corresponding to the average density of the digital tomographic survey (25), or a density distribution derived from the digital tomographic survey (25).

9. Method for producing an anatomical prosthesis made of flexible synthetic material via computer-aided three-dimensional design according to any one of Claims 3 to 8, wherein a finishing step makes it possible to reproduce, after moulding the anatomical prosthesis (2), the pigmentation of the various regions of the volume of said anatomical prosthesis (2) identically to the anatomical part to be replaced by the anatomical prosthesis (2) based on the digital colorimetric survey, and/or centimetre-scale photographic survey.

10. Method for producing an anatomical prosthesis made of flexible synthetic material via computer-aided three-dimensional design according to either of Claims 3 and 4, wherein the anatomical prosthesis (2) is produced by additive manufacturing based on:
- said first data so that the shape of the volume of said anatomical prosthesis (2) is identical to the shape of the anatomical part to be replaced by said anatomical prosthesis (2);
- said second data so that the visual appearance of said anatomical prosthesis (2) is identical to the visual appearance of the removed anatomical part; and/or
- said third data so that the density distribution in said anatomical prosthesis (2) is identical to the density distribution in the anatomical part to be replaced by said digital prosthesis (2).

11. Method for producing an anatomical prosthesis made of flexible synthetic material via computer-aided three-dimensional design according to any of Claims 3 to 10, wherein the flexible synthetic material used is a biomedical silicone the bulk of which is tinted to reproduce the colours to the tone and saturation level required by the digital colorimetric survey.

12. Computer program product comprising instructions that, when they are executed by a computer, cause the computer to implement the method according to any of Claims 1 to 11.

13. Computer-readable data medium on which is recorded the computer program product according to Claim 12.

14. Signal of a data medium, carrying the computer program product according to Claim 12.
